# EUROPEAN PATENT SPECIFICATION

(11) **EP 2 150 309 B1**
(45) Date of publication and mention of the grant of the patent: **11.09.2013**
(21) Application number: 07748135.6
(22) Date of filing: 15.05.2007
(51) Int. Cl.: A61N 1/05

(54) **MEDICAL IMPLANTABLE LEAD WITH PIVOTING SEGMENTS**
MEDIZINISCHE IMPLANTIERBARE LEITUNG MIT SCHWENKSEGMENTEN
CONDUCTEUR MÉDICAL IMPLANTABLE À SEGMENTS PIVOTANTS

(43) Date of publication of application: 10.02.2010
(73) Proprietor: St. Jude Medical AB, 175 84 Järfälla (SE)
(72) Inventor: HILL, Rolf, S-175 55 Järfälla (SE); STEGFELDT, Olof, S-138 57 Älta (SE)
(74) Representative: Sjölander, Henrik
(86) International application number: PCT/SE2007/000471
(87) International publication number: WO 2008/140365

(56) References cited:
- US-A- 4 506 679
- US-A- 4 913 164
- US-A- 4 957 118
- US-A- 5 300 107
- US-A- 5 545 206
- US-A- 5 755 767
- US-A1- 2004 133 259
- US-A1- 2005 038 491
- US-A1- 2006 190 068
- US-A1- 2007 049 980
- US-A1- 2007 050 003
- US-A1- 2007 073 370
- US-B1- 6 684 109

## Description

The invention relates to a medical implantable lead, which is adapted to be attached with a distal end to tissue inside a human or animal body, wherein the distal end is variable between an introducing state, when the distal end has a minimum surface area, and a mounting state when the surface area of the distal end is enlarged in relation to its minimum surface area.

### Background of the invention

Medical implantable leads of various kinds and for various applications, e.g. for monitoring and controlling the heart in a human or animal body by means of a pacemaker, tend to become smaller and smaller in cross section. A medical implantable lead for pacemaker applications, for example, can have a diameter of less than 2 mm. This is advantageous in one aspect, since then the lead will be more flexible and take up less space. However, there are also risks with leads having a too small cross sectional dimension. In many cases the leads are namely adapted to be mounted to an organ inside the body, e.g. a heart wall, with its end surface abutting the organ and held by means of for example a helix, which is screwed into the organ. If the cross sectional dimension of the lead is too small in relation to its stiffness, it is a risk that the lead tip will perforate the organ during mounting of the lead and cause bleeding. This risk exists on the one hand when the distal end is pressed against the organ but before the lead is actually attached to it. In case the lead is provided with a rotatable helix for attaching to the organ, this risk also exists when the helix is screwed into the organ such that the distal end is drawn into the tissue by means of the rotating action of the helix. When the lead is attached to e.g. a heart, which performs large movements during function, there is also a risk that the heart wall will be perforated or injured during the course of a longer period of time when the lead is attached, due to abrasion or the like, if the lead is made with a too small cross sectional dimension in relation to its stiffness.

From US 2007/0050003 A1, is known a medical implantable lead, which in one embodiment (Figs 7A-C) is provided with an elastic sleeve in a distal end which protrudes a distance beyond a distal end of the rest of the lead and which, when abutting the distal end against tissue or attaching the lead to the tissue by means of screwing a helix into the tissue, will buckle and expand to a diameter that is larger than the diameter of the distal end of the lead itself. However, there are several disadvantages with a medical implantable lead of this kind. To accomplish buckling of the sleeve, it is required a rather large force having to effect that, when the lead is mounted with its distal end in abutment against tissue, the sleeve will affect the lead with a force striving to push the lead from and disengage the helix from the tissue. This is not satisfactory since it may lead to that the helix might slip out of engagement with the tissue during long term usage due to movements in the tissue, e.g. a heart. To alleviate this effect, one possible solution would be to make the sleeve extremely elastic and flexible, but this would also have to effect that the contribution of the sleeve for preventing perforation into the tissue, would be reduced correspond-ingly. It would also have the effect that the risk for unintentional buckling of the sleeve, and hence an increased lead tip area, when introducing the lead through e.g. a narrow vein will increase, which might make it impossible to insert the lead.

US-A-4 506 679 discloses endocardial electrodes having outwardly extending tines located near an electrode tip.

US-B-6 684 109 discloses an implantable cardioversion and defibrillation lead for applying electrical energy to the heart, wherein the defibrillation electrodes are flexible arms that are swept proximally or distally during introduction and when the electrode arms are extended out from the distal end they deploy into a radially extended position.

US-A-4 957 118 discloses a pacemaker electrode lead having a tine assembly in which struts are disposed in pairs and pivotally connected to a respective tine via a pin.

### Summary of the invention

It is an object of the invention to provide an improved medical implantable lead by which the risk of per-foration into tissue is eliminated or reduced and yet can ensure secure attachment to tissue. At least this object is achieved by a medical implantable lead according to claim 1.

The basis of the invention is the insight that the above object may be achieved by providing the medical implantable lead with means for varying the surface area of the distal end of the lead, such that the surface area has a minimum when introducing the lead into the body. Once the lead is localised inside the body, the surface area of the distal end can be enlarged when abutting the distal end against an organ or other tissue.

According to the invention, the enlargement of the surface area is accomplished by mechanically pivoting of material portions at the distal end. More precisely, the distal end of the lead is provided with several pivoting segments distributed around its circumference, each of the pivoting segments being pivotally hinged about a pivot axis directed substantially tangential in relation to the lead. According to the invention, the pivot axis is located on the inside close to the centre of each pivoting segment. Each pivoting segment is pivotal about the pivot axis between a first, introducing position, when each pivoting segment is rotated backward/outward to a position being in close contact with the outer surface of the lead and in parallel or in a small angle to the longitudinal axis of the lead, and a second, mounting position when each pivoting segment is rotated to a position essentially perpendicular to the longitudinal axis of the lead. With a medical implantable lead arranged in this way, no force is required to assume the mounting state with an enlarged surface area, also having the effect that no force is acting on the lead from the pivoting segments striving to push the lead away from the tissue. There is also no risk that the mounting state, with an enlarged surface area, is unintentionally assumed during introduction of the lead through a vein or the like.

In the hereinafter described and illustrated embodiment, the articulated hinge between the distal end of the lead and the pivoting segments, is accomplished by a sleeve of an elastic material, e.g. silicon plastics, which covers the outside of the lead, and the pivoting segments are formed of the same material as and in one unitary piece with the sleeve, and such that they are connected together by a thin tongue of the elastic material having the effect that the pivotal action of the pivoting segments are achieved by elastic deformation of the tongue. However, it also would be possible to form the pivoting segments as separate objects, in relation to the rest of the lead, and to connect them via a mechanical hinge to the lead, e.g. by snap fitting.

Preferably, the lead according to the invention also has an extracting state in which each pivoting segment is rotated forward/inward such that an outer edge of each pivoting segment is positioned further in a distal direction than an inner edge. In this way the surface area of the distal end will be reduced in relation to the mounting state to facilitate extraction of the medical implantable lead from the human or animal body.

It is to be understood that the medical implantable lead may be modified in many different ways in relation to the hereinafter described and illustrated embodiment. E.g. the ring segments may have many different shapes than the herein showed ring segment shape, though it is normally advantageous if the segments have a generally flat shape.

### Brief description of the drawings

An embodiment of the invention will now be described by way of example by reference to the accompanying drawings, in which:
- Fig 1: is a perspective view of a distal end of a medical implantable lead, according to the in-vention, in a mounting state when the pivoting segments have been rotated to a position sub-stantially perpendicular to the longitudinal axis of the lead;
- Fig 2: is a perspective view of the outermost distal end of the lead in fig 1 in an introducing state when the pivoting segments have been ro-tated backward/outward;
- Fig 3: is a perspective view of the outermost distal end of the lead in figs 1 and 2 in an extract-ing state when the pivoting segments have been rotated forward/inward;
- Fig 4: is an enlarged longitudinal section through the distal end of the lead in figs 1-3;
- Fig 5: is a longitudinal section through the distal end of the lead when being introduced through a vein;
- Fig 6: is a longitudinal section of the lead according to fig 5 in a mounted state with the distal end abutting against a heart wall; and
- Fig 7: is a longitudinal section according to fig 5 and 6 with the lead in an extracting state be-ing extracted out from the body through a vein;

### Detailed description of an embodiment of the invention

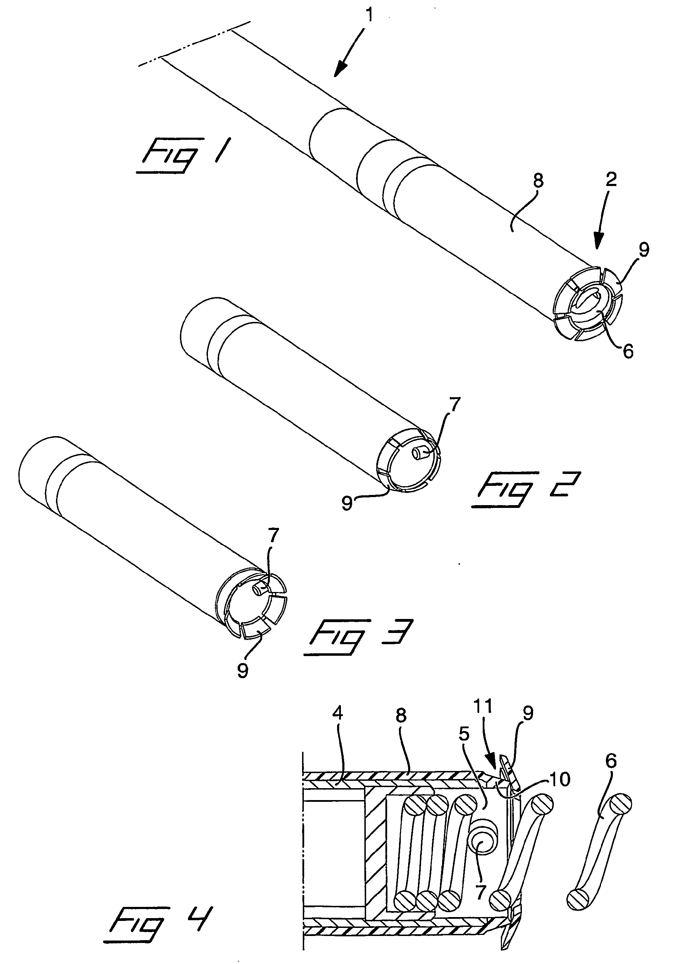

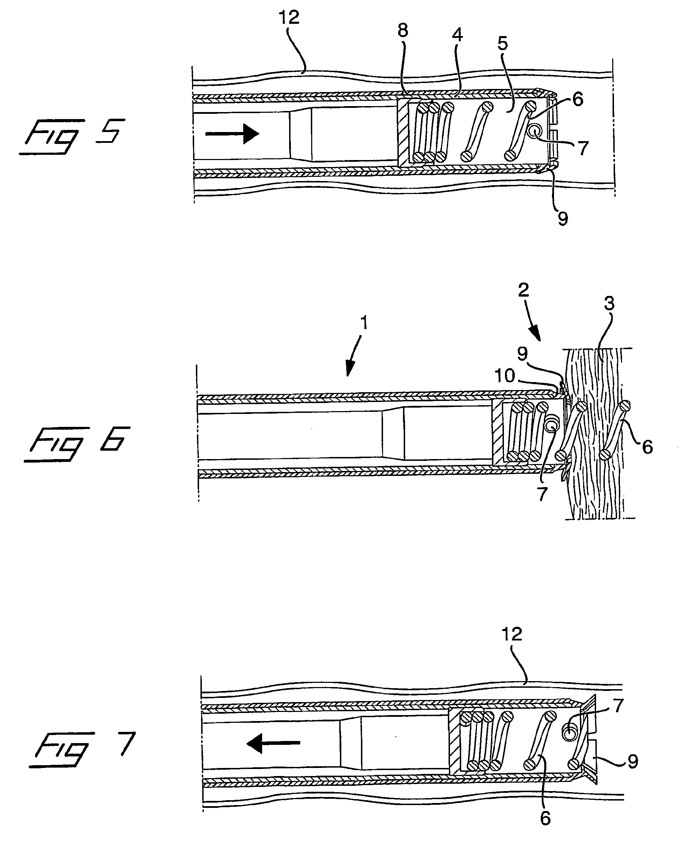

The medical implantable lead according to an embodiment of the invention and being illustrated in the drawings, is adapted to be used in connection with a pacemaker or an implantable cardioverter defibrillator. Accordingly, the lead comprises a considerably long electrical lead 1, of which only a distal end is shown in the drawings. A pacemaker is adapted to be connected to a proximal end of the lead, whereas a distal end 2 is adapted to be attached to a heart wall 3. As is best seen in figs 4-7, the lead is, in a distal end, provided with a rigid tubular header 4 of a metal or plastics. In a distal end of a bore 5 in the header, a helix 6 is rotatably arranged. The helix can be rotated from the proximal end of the lead, by suitable means known in the art but not shown in the drawings, such as a stylet or a coil. Besides being rotatably, the helix 6 is also displaceable in an axial direction in the header 4. This is achieved by the helix winding being in engagement with a post 7 on the inside of the header, such that when rotating the helix, it will also be displaced in the axial direction of the lead. This is illustrated in fig 5, where the helix 6 is screwed in and completely accommodated within the header bore 5, and in fig 6 where the helix is screwed out such that a distal portion of the helix protrudes a distance out from the header and extended into the tissue of a heart wall 3. On the outside of the header the lead is provided with a cover or sleeve 8 of a resilient material, e.g. of silicone.

As is illustrated, the distal end of the medical implantable lead according to the invention, is provided with several pivoting segments 9. From the enlargement of fig 4, appears that the pivoting segments are formed of the same material and in a unitary piece with the elastic sleeve 8 on the outside of the header. Each pivoting segment is formed as a ring segment, having a generally flat shape, and is pivotal by elastic deformation of a tongue 10, which connects the pivoting segment 9 with the elastic sleeve 8. Accordingly, each segment is individually pivotal in relation to the header 4. The illustrated lead is provided with six separate segments and it is believed that at least four segments are required for a proper function.

From fig 4 is also evident that the pivoting segments 9 are connected to the elastic sleeve 8 in an area near the middle of each segment but displaced towards the inner periphery of the tubular header 4, and the sleeve is provided with a recessed or bevelled portion 11 on the outer side of the distal end. The latter is due to the fact that preferably no part of the segments should protrude beyond the outer boundaries of the lead in the introducing state according to fig 5. The recessed portion 11 has a depth that is at least as large as the thickness of the segments such that the segments can be accommodated in the recessed area in the introducing state. In this way the pivoting segments 9 may be pivoted backward/outward and accommodated in the recessed portion 11 such that essentially no part of the pivoting segments protrude outside the outermost periphery of the lead when the lead is in the introducing state according to fig 2 and 5.

Preferably, the initial position of the pivoting segments 9, when no forces are effecting them, is as is illustrated in fig 1, i.e. the pivoting segments are positioned with their principal plane substantially perpendicular to the longitudinal axis of the lead. Accordingly, the segments will adopt this position as soon as the distal end comes into a cavity in a body. This position will also be adopted as soon as the distal end of the lead comes into contact with a surface, e.g. a wall 3 of a heart, when the helix is rotated and screwed into tissue for attaching the lead to the tissue. In this way the surface area of the lead is increased such that the risk for penetration of the lead into tissue is reduced. When the lead is introduced into a human or animal body, through a vein 12 or the like, the pivoting segments assume an introducing state with the segments pivoted backward/outward with only a small angle in relation to the longitudinal axis of the lead, as is illustrated in fig 2 and 5.

When extracting the lead from the body, the pivoting segments 9 will adopt the position illustrated in fig 7. Namely, when the lead encounter resistance from e.g. the inside of a vein 12, the outer edges of the segments will slide against the walls of the vein 12 and be pivoted forward/inward such that the outer edges of the segments will be positioned further in the distal direction in relation to the inner edges. This situation may occur several years after implanting of the lead into the body and by that time, the distal end can be more or less overgrown by tissue. In this way the lead can be extracted with a reduced risk for damage to or entangle in tissue.

When implanting the lead into a human or animal body, e.g. by introducing it through a vein 12, the lead is in the introducing state as is shown in fig 2 and 5. In this state the helix is screwed into and accommodated in the header bore 5, which prevents the helix from penetration into tissue during introduction. When the lead has been introduced into the body and the distal end of the lead, with the pivoting segments 9 in a mounting state substantially perpendicular to the longitudinal axis, is bearing against tissue at a location to be mounted in, the helix 6 is caused to rotate by means of a not shown rotary transmitting means, such as a stylet or a coil, by means of which a rotary movement can be transmitted from a proximal end to the distal end of the lead. When rotating the helix, its tip will penetrate into the tissue 3 and attach the lead securely to the tissue, as shown in fig 6.

## Claims

1. A medical implantable lead, which is adapted to be attached with a distal end (2) to tissue (3) inside a human or animal body, wherein the distal end is variable between an introducing state, when the distal end has a minimum surface area, and a mounting state when the surface area of the distal end is enlarged in relation to its minimum surface area, wherein the medical implantable lead (1) in the distal end (2) comprises several pivoting segments (9), which each is pivotally hinged about a pivot axis directed substantially tangential in relation to the lead and located on the inside of and close to the centre of each pivoting segment, wherein each pivoting segment is pivotal about the pivot axis between an introducing state, when each pivoting segment is rotated to a position in parallel or in a small angle to the longitudinal axis of the lead, and a mounting state, when each pivoting segment is rotated to a position essentially perpendicular to the longitudinal axis of the lead, and wherein each pivoting segment is arranged to be pivoted about said pivot axis when said distal end contacts an organ or other tissue during introduction of said lead so as to adopt said mounting state and enlarge the surface area of the distal end.

2. A medical implantable lead according to claim 1, **characterized in that** the pivoting segments (9) are formed of the same material and in a unitary piece with an elastic sleeve (8) on the outside of the lead (1), and the pivoting segments are pivotally hinged by means of an elastic tongue (10) between the elastic sleeve and the segments.

3. A medical implantable lead according to claim 1 or 2, **characterized in that** the pivoting segments are formed as ring segments (9).

4. A medical implantable lead according to any of the preceding claims, **characterized in that** the distal end of the lead (1) is provided with a recessed or bevelled portion (11) on an outer side for accommodating the pivoting segments (9) in the introducing state.

5. A medical implantable lead according to any of the preceding claims, **characterized in that** the pivoting segments (9) are pivotal hinged to the lead (1) at a position localized towards an inner periphery of a tubular header (4).

6. A medical implantable lead according to any of the preceding claims, **characterized in that** it is provided with at least four pivoting segments (9).

7. A medical implantable lead according to any of the preceding claims, **characterized in that** it also has an extracting state in which the surface area of the distal end is reduced in relation to the mounting state to facilitate extraction of the medical implantable lead from the human or animal body.

8. A medical implantable lead according to claim 8, **characterized in that** the extracting state is achieved by rotating the pivoting segments (9) forward/inward such that said outer edge of each pivoting segment is positioned further in a distal direction than said inner edge.

## Patentansprüche

1. Medizinische implantierbare Leitung, die so ausgelegt ist, dass sie mit einem distalen Ende (2) an Gewebe (3) in einem menschlichen oder tierischen Körper angebracht wird, wobei das distale Ende veränderlich ist zwischen einem Einführzustand, wenn das distale Ende einen minimalen Flächeninhalt aufweist, und einem Befestigungszustand, wenn der Flächeninhalt des distalen Endes bezogen auf seinen minimalen Flächeninhalt vergrößert ist, wobei die medizinische implantierbare Leitung (1) am distalen Ende (2) mehrere Schwenksegmente (9) umfasst, wobei jedes drehgelenkig um eine Schwenkachse angebracht ist, die im Wesentlichen tangential bezogen auf die Leitung ausgerichtet ist und sich an der Innenseite und in der Nähe des Mittelpunkts jedes Schwenksegments befindet, wobei jedes Schwenksegment um die Schwenkachse zwischen einem Einführzustand, wenn jedes Schwenksegment in eine Position parallel oder unter einem kleinen Winkel zur Längsachse der Leitung gedreht ist, und einem Befestigungszustand, wenn jedes Schwenksegment in eine Position im Wesentlichen senkrecht zu der Längsachse der Leitung gedreht ist, drehgelenkig bewegbar ist, und wobei jedes Schwenksegment so angeordnet ist, dass es um die Schwenkachse geschwenkt wird, wenn das distale Ende ein Organ oder anderes Gewebe während des Einführens der Leitung berührt, um den Befestigungszustand anzunehmen und den Flächeninhalt des distalen Endes zu vergrößern.

2. Medizinische implantierbare Leitung nach Anspruch 1, **dadurch gekennzeichnet, dass** die Schwenksegmente (9) aus demselben Material und einteilig mit einer elastischen Hülle (8) an der Außenseite der Leitung (1) gebildet sind und die Schwenksegmente mittels einer elastischen Zunge (10) zwischen der elastischen Hülle und den Segmenten drehgelenkig angebracht sind.

3. Medizinische implantierbare Leitung nach Anspruch 1 oder 2, **dadurch gekennzeichnet, dass** die Schwenksegmente als Ringsegmente (9) geformt sind.

4. Medizinische implantierbare Leitung nach einem der vorhergehenden Ansprüche, **dadurch gekennzeichnet, dass** das distale Ende der Leitung (1) mit einem vertieften oder schrägen Abschnitt (11) an einer Außenseite zum Aufnehmen der Schwenksegmente (9) im Einführzustand versehen ist.

5. Medizinische implantierbare Leitung nach einem der vorhergehenden Ansprüche, **dadurch gekennzeichnet, dass** die Schwenksegmente (9) an einer Position, die sich in Richtung eines Innenumfangs eines röhrenförmigen Kopfteils (4) befindet, drehgelenkig an der Leitung (1) angebracht sind.

6. Medizinische implantierbare Leitung nach einem der vorhergehenden Ansprüche, **dadurch gekennzeichnet, dass** sie mit mindestens vier Schwenksegmenten (9) versehen ist.

7. Medizinische implantierbare Leitung nach einem der vorhergehenden Ansprüche, **dadurch gekennzeichnet, dass** sie auch einen Entnahmezustand aufweist, in dem der Flächeninhalt des distalen Endes bezogen auf den Befestigungszustand verkleinert ist, um das Herausziehen der medizinischen implantierbaren Leitung aus dem menschlichen oder tierischen Körper zu erleichtern.

8. Medizinische implantierbare Leitung nach Anspruch 8, **dadurch gekennzeichnet, dass** der Entnahmezustand erreicht wird, indem die Schwenksegmente (9) nach vorn/innen gedreht werden, sodass der Außenrand jedes Schwenksegments weiter in einer distalen Richtung platziert ist als der Innenrand.

## Revendications

1. Conducteur médical implantable, qui est adapté pour être fixé avec une extrémité distale (2) à un tissu (3) dans un corps humain ou animal, l'extrémité distale étant réglable entre un état d'introduction, quand l'extrémité distale présente une superficie minimale, et un état de montage quand la superficie de l'extrémité distale est agrandie par rapport à sa superficie minimale, le conducteur médical implantable (1) dans l'extrémité distale (2) comprenant plusieurs segments pivotants (9), dont chacun est articulé pivotant autour d'un axe de pivotement orienté essentiellement tangentiel par rapport au conducteur et situé sur l'intérieur et près du centre de chaque segment pivotant, chaque segment pivotant pouvant pivoter autour de l'axe de pivotement entre un état d'introduction, quand chaque segment pivotant est tourné dans une position parallèle ou à un angle faible par rapport à l'axe longitudinal du conducteur, et un état de montage, quand chaque segment pivotant est tourné dans une position essentiellement perpendiculaire à l'axe longitudinal du conducteur, et chaque segment pivotant étant agencé pour être pivoté autour dudit axe de pivotement quand ladite extrémité distale touche un organe ou d'autres tissus pendant l'introduction dudit conducteur de façon à adopter ledit état de montage et agrandir la superficie de l'extrémité distale.

2. Conducteur médical implantable selon la revendication 1, **caractérisé en ce que** les segments pivotants (9) sont formés avec le même matériau et en une pièce unique avec une gaine élastique (8) sur l'extérieur du conducteur (1), et les segments pivotants sont articulés pivotants au moyen d'une languette élastique (10) entre la gaine élastique et les segments.

3. Conducteur médical implantable selon la revendication 1 ou 2, **caractérisé en ce que** les segments pivotants sont sous forme de segments annulaires (9).

4. Conducteur médical implantable selon l'une quelconque des revendications précédentes, **caractérisé en ce que** l'extrémité distale du conducteur (1) est dotée d'une partie évidée ou biseautée (11) sur une face extérieure pour loger les segments pivotants (9) dans l'état d'introduction.

5. Conducteur médical implantable selon l'une quelconque des revendications précédentes, **caractérisé en ce que** les segments pivotants (9) sont articulés pivotants sur le conducteur (1) dans une position située vers une périphérie intérieure d'un connecteur tubulaire (4).

6. Conducteur médical implantable selon l'une quelconque des revendications précédentes, **caractérisé en ce qu'**il est doté d'au moins quatre segments pivotants (9).

7. Conducteur médical implantable selon l'une quelconque des revendications précédentes, **caractérisé en ce qu'**il présente également un état d'extraction dans lequel la superficie de l'extrémité distale est réduite par rapport à l'état de montage pour faciliter l'extraction du conducteur médical implantable du corps humain ou animal.

8. Conducteur médical implantable selon la revendication 8, **caractérisé en ce que** l'état d'extraction est obtenu en faisant tourner les segments pivotants (9) vers l'avant/l'intérieur de sorte que ledit bord extérieur de chaque segment pivotant est positionné plus loin dans une direction distale que ledit bord intérieur.
